# EUROPÄISCHE PATENTSCHRIFT

(11) **EP 3 700 881 B1**
(45) Veröffentlichungstag und Bekanntmachung des Hinweises auf die Patenterteilung: **24.01.2024**
(21) Anmeldenummer: 17791057.7
(22) Anmeldetag: 25.10.2017
(51) Int. Cl.: C07C 45/34, C07C 49/587

(54) **VERFAHREN ZUR SYNTHESE EINES UNGESÄTTIGTEN MAKROZYKLISCHEN KETONS**
METHOD FOR SYNTHESISING AN UNSATURATED MACROCYCLIC KETONE
PROCÉDÉ DE SYNTHÈSE D'UNE CÉTONE MACROCYCLIQUE INSATURÉE

(43) Veröffentlichungstag der Anmeldung: 02.09.2020
(73) Patentinhaber: Symrise AG, 37603 Holzminden Niedersachsen (DE)
(72) Erfinder: BRUNZEL, Tom, 18057 Rostock (DE); KÖCKRITZ, Angela, 12437 Berlin (DE); MARTIN, Andreas, 12524 Berlin (DE); TREVINO, Diego, A. Jaime, 4150 Dormagen (DE)
(74) Vertreter: Fabry, Bernd
(86) Internationale Anmeldenummer: PCT/EP2017/077298
(87) Internationale Veröffentlichungsnummer: WO 2019/081009

(56) Entgegenhaltungen:
- EP-A1- 2 364 965
- US-A1- 2014 194 604
- VILLEMIN D ET AL: "SUPPORTED METALATED PHTHALOCYANINE AS CATALYST FOR OXIDATION BY MOLECULAR OXYGEN. SYNTHESIS OF QUINONES AND CARBONYL COMPOUNDS", SYNTHETIC COMMUNICATIONS, TAYLOR & FRANCIS INC, PHILADELPHIA, PA; US, Bd. 32, Nr. 10, 1. Januar 2002 (2002-01-01), Seiten 1501-1515, XP001115801, ISSN: 0039-7911, DOI: 10.1081/SCC-120004139
- RYAN J. DELUCA ET AL: "Wacker-Type Oxidation of Internal Alkenes using Pd(Quinox) and TBHP", JOURNAL OF ORGANIC CHEMISTRY, Bd. 78, Nr. 4, 15. Februar 2013 (2013-02-15), Seiten 1682-1686, XP055479024, ISSN: 0022-3263, DOI: 10.1021/jo302638v

## Beschreibung

### GEBIET DER ERFINDUNG

Das Gebiet der Erfindung ist die Herstellung von ungesättigten makrozyklischen Monoketonen aus einem makrozyklischen Dien in einer einstufigen Oxidation in Gegenwart eines Oxidationsmittels, eines Palladium(II)-Salzes und/oder eines Palladium(II)-Komplexes und eines Lösungsmittels.

### TECHNOLOGISCHER HINTERGRUND

Ketone sind als Lösungsmittel und chemische Rohstoffe geeignet und werden daher in verschiedenen Bereichen eingesetzt. Solche Ketone werden im Allgemeinen durch zweistufige Reaktionsverfahren hergestellt, bei denen ein durch Hydratisieren eines Olefins erzeugter Alkohol dehydriert wird. Mittlerweile sind als einfachere Verfahren auch einstufige Reaktionsverfahren bekannt, bei denen ein Olefin direkt oxidiert wird.

Zweistufige Verfahren zur Herstellung von makrozyklischen Monoketonen sind aus dem Stand der Technik bekannt. Die technische Synthese von makrozyklischen Ketonen gelingt beispielsweise über eine selektive Monoepoxidierung und Umlagerung des Epoxids zum Keton in Gegenwart von Alkali- oder Erdalkalihalogeniden, wie in B. D. Mookherjee, R. W. Trenkle, R. R. Patel, J. Org. Chem. 36 (1971), p3266 beschrieben. Eine Reduktion der Epoxygruppe zu einer Hydroxygruppe, gefolgt von der anschließenden Oxidation zum Keton wird beispielsweise in US 3718696 A (IFF) beschrieben.

Das Wacker-Verfahren, unter Verwendung eines PdCl₂ / CuCl₂-Katalysators, ist als eines der Verfahren zur direkten Oxidation eines Olefins bekannt. Dieses Verfahren ist wirksam für die Oxidation von terminalen Olefinen mit jeweils einer Kohlenstoff-Kohlenstoff-Doppelbindung (nachfolgend als "C = C-Bindung" abgekürzt) an einem Ende eines Moleküls davon. Jedoch erzielt dieses Verfahren nur eine geringe Reaktivität, wenn es für die Oxidation von internen Olefinen verwendet wird, die jeweils eine C = C-Bindung an einer anderen Position als ihren Enden aufweisen. Dieses Verfahren erzielt ebenso bei einer Erhöhung der Anzahl der Kohlenstoffatome des Ausgangsmaterials keine zufriedenstellenden Ergebnisse, da die Reaktionsgeschwindigkeit deutlich gesenkt wird. Aus diesem Grund ist auf dem industriellen Gebiet die Verwendung des Wacker-Verfahrens nur auf die Herstellung von niederen Carbonylverbindungen wie Acetaldehyd und Aceton beschränkt, die durch Oxidation von niederen terminalen Olefinen erhalten werden.

### STAND DER TECHNIK

Kaneda *et al.* entwickelte eine Methode, welche kein Cooxidans für die Reoxidation von Pd(0) benötigt, sondern in welcher Sauerstoff direkt von Pd(0) zu Pd(II) oxidiert. Hierbei wird ein Dimethylacetamid/H₂O-Gemisch bei 80 °C verwendet (T. Mitsudome, T. Umetani, N. Nosaka, K. Mori, T. Mizugaki, K. Ebitani, K. Kaneda, ANGEW. CHEMIE INT Ed. 45 (2006) 481**).**

Dieses Verfahren gestattet auch die Oxidation linearer interner Olefine und cyclischer Olefine, wie beispielsweise Cyclohexen (T. Mitsudome, K. Mizumoto, T. Mizugaki, K. Jitsukawa, K. Kaneda, ANGEW. CHEMIE INT Ed. 49 (2010) 1238). Erfolgreich waren hierbei jedoch nur chlorhaltige Palladium-Katalysatoren. Eigene Untersuchungen zeigten, dass aus makrozyklischen Olefinen und Dienen als Substrat nach dieser Methode, auch bei Verwendung eines Cooxidationsmittels, nur unzureichende Ausbeuten an den gewünschten Ketonen erzielt werden.

Aus SYNTHETIC COMMUNICATIONS 2002, 32 (10), p1501-115 ist die Oxidation von Carbonylverbindungen bekannt, wobei als Katalysatoren Phthalocyanine auf Trägern eingesetzt werden.

In J ORG CHEM 2013, 78(4), p1682-1686 wird eine Wacker-Oxidation von innenständigen Olefinen mit Palladium-Katalysatoren beschrieben, bei denen es sich allerdings um spezielle Chinolin-Ligand-Systeme handelt. Die Reaktion erfordert zudem einen bestimmten Co-Katalysator, nämlich tert.-Butylhydroperoxid.

Gegenstand der EP 2364965 A1 (KANEDA) ist ein Verfahren zur Herstellung von Ketonen, bei dem man ungesättigte Olefine (z.B. Cycloocten) mit Sauerstoff in Gegenwart von Pd-Katalysatoren in amidischen Lösungsmitteln oxidiert. Als Pd-Katalysatoren kommen die Halogenide sowie deren Komplexe mit den amidischen Lösungsmitteln in Betracht, jedoch keine Pd-Ligand-Systeme.

Eine weitere Variation der Wacker-Oxidation von innenständigen Olefinen unter Verwendung kationischer Palladiumkomplexe wird in der US 2014 0194604 AA (CALTECH) beschrieben. Hierbei werden jedoch keine makrozyklischen Diene als Ausgangssubstrat verwendet. Bei den Katalysatoren handelt es sich um Palladiumsalze, wie z.B. Palladiumacetat.

Ebenso beschreibt die WO 2010 061807 A1 (NIPPON OIL) eine Abwandlung des Wacker-Verfahrens in welcher auch makrozyklische Diene als Ausgangssubstrat zum Einsatz kommen.

Makrozyklische Diene bilden mit Pd(II)-Spezies/Verbindungen einen Chelatkomplex. Durch die räumliche Anordnung des Liganden und seine Flexibilität wird jedoch die weitere Koordination der reagierenden Komponenten behindert, wodurch deutlich die Aktivität und Selektivität der Reaktion eingeschränkt wird. Dies ist eine der Ursachen, warum solche makrozyklische Diene in den aus dem Stand der Technik bekannten Verfahren nur sehr schlecht reagieren. Zudem wird dies dadurch bestärkt, dass diese sehr unpolaren makrozyklischen Diene nur eine begrenzte Löslichkeit in polaren Lösungsmitteln aufweisen. Zufriedenstellende Ausbeuten lassen sich folglich mit den aus dem Stand der Technik bekannten Verfahren nicht erzielen.

### AUFGABE DER ERFINDUNG

Aufgabe der vorliegenden Erfindung war es daher, ein Verfahren bereitzustellen, dasdie Nachteile des Stands der Technik überwindet. Konkret bestand die Aufgabe darin, ein Verfahren zu entwickeln, welches eine einfache, material- und energiesparende und somit nachhaltige Prozessführung ermöglicht und durch welches gleichzeitig wirtschaftliche Ausbeuten erzielt werden.

### BESCHREIBUNG DER ERFINDUNG

Diese Aufgabe wird gelöst durch den Gegenstand der vorliegenden Erfindung welche ein Verfahren zur Herstellung von ungesättigten makrozyklischen Monoketonen betrifft, umfassend die folgenden Schritte:
(a) Bereitstellung von makrozyklischen Dienen mit einer Ringgröße von mindestens 9 Kohlenstoffatomen;
(b) Inkontaktbringen der Ausgangsstoffe aus Schritt (a) mit
   (b1) einem Palladium(II)-Salz und/oder einem Palladium(II)-Komplex; sowie
   (b2) einem Bidentatligand, der N,N-, N,O-, N,S- oder O,O-Donoratome enthält, und
   (b3) einem Oxidationsmittel; und
   (b4) einem Lösungsmittel; und gegebenenfalls
   (b5) einem Co-Katalysator; und gegebenenfalls
   (b6) einer Säure.

Überraschenderweise wurde gefunden, dass mittels des oben genannten Verfahrens ungesättigte makrozyklische Monoketone in deutlich höheren Ausbeuten hergestellt werden können. Ebenfalls ist eine kommerzielle Verwendung der entstehenden Isomere möglich, was das Verfahren äußerst nachhaltig und effizient gestaltet.

Ein weiterer Vorteil der Erfindung ist es, dass die makrozyklischen Diene durch das erfindungsgemäße Verfahren derart effektiv umgesetzt werden, dass selbst, wenn die makrozyklischen Diene als Gemisch von E,E-, E,Z- und Z,Z-Isomeren vorliegen, die jeweils eine unterschiedliche Reaktivität aufweisen, diese selektiv zu einem Monoketon oxidiert werden können, ohne dass unerwünschte Mengen an Diketonen entstehen.

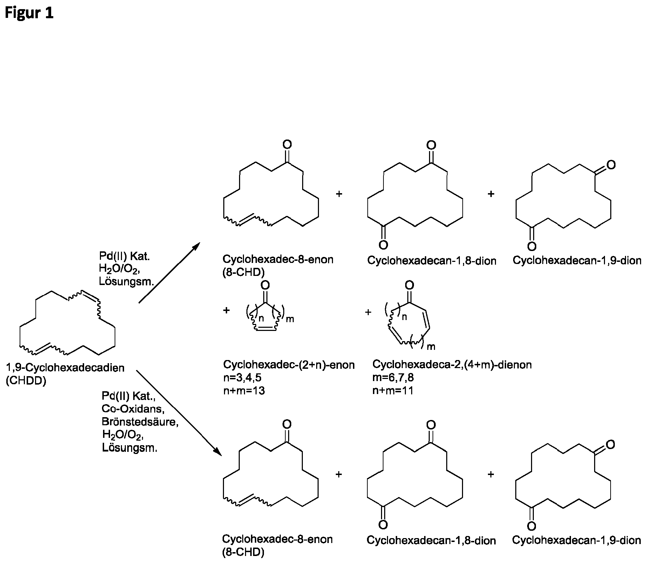

**Fig. 1** veranschaulicht beispielhaft den Ablauf des erfindungsgemäßen Herstellungsverfahrens. Diese Abbildung stellt jedoch nur ein Beispiel dar und soll keinesfalls einschränkend sein.

### Makrozyklische Diene

Bevorzugt enthält das makrozyklische Dien (a) zwei nichtkonjugierte Doppelbindungen. In einer bevorzugten Ausführungsform der Erfindung hat das makrozyklische Dien eine Ringgröße von 9 bis 30 Kohlenstoffatomen, bevorzugt von 12 bis 18 Kohlenstoffatomen und besonders bevorzugt von 16 Kohlenstoffatomen. In eines besonders bevorzugten Ausführungsform ist das makrozyklische Dien (a) 1,9-Cyclohexadecadien (CHDD).

In einer weiteren bevorzugten Ausführungsform des erfindungsgemäßen Verfahrens ist das verwendete makrozyklische Dien (a) ein Gemisch aus verschiedenen Stereoisomeren. In einer besonders bevorzugten Ausführungsform des erfindungsgemäßen Verfahrens ist das makrozyklische Dien (a) 1,9-Cyclohexadecadien (CHDD) und liegt als Gemisch von E,E-, E,Z- und Z,Z-Isomeren vor.

Bevorzugt wird durch das erfindungsgemäße Herstellungsverfahren Cyclohexadec-8-enon (8-CHD) hergestellt. In einer besonders bevorzugten Ausführungsform der Erfindung wird als das makrozyklische Dien (a) CHHD verwendet wobei durch Anwendung des erfindungsgemäßen Herstellungsverfahrens 8-CHD erhalten wird.

### Palladiumsalze und Palladiumkomplexe

Das Palladium(II)-Salz und/oder der Palladium(II)-Komplex (b1) werden in einer ersten Ausführungsform der erfindungsgemäßen Verfahrens ausgewählt aus der Gruppe bestehend aus Palladiumchlorid, Palladiumbromid, H₂PdCl₄, Li₂PdCl₄, Na₂PdCl₄, K₂PdCl₄, (NH₄)₂PdCl₄, Palladiumacetat, Palladiumtrifluoracetat, Palladiumbenzoat, Tetrakis(acetonitril)palladium(II) tetrafluoroborat, Tetrakis(acetonitril)palladium (II) Bis(trifluoromethansulfonat), Palladiumnitrat und/oder Palladiumsulfat.

Weiterhin ist eine erste Ausführungsform des erfindungsgemäßen Verfahrens dadurch gekennzeichnet, dass das Palladium(II)-Salz und/oder der Palladium(II)-Komplex (b1) mit dem Bidentatligand (b2) ebe eine Palladium-Verbindung der Formel (I) und/oder der Formel (II) bilden 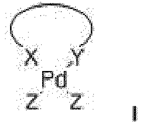
- wobei X und Y jeweils unabhängig voneinander für N,N, O,N oder O,O steht; und
- wobei Z jeweils unabhängig voneinaner für ein Halogen, Acetat, Trimethylacetat, Trifluormethylacetat, MeCN, PhCN, NO₂, NO, Nitrat, Nitrit oder Sulfat steht; 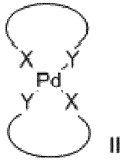
- wobei X und Y jeweils unabhängig voneinander für O,O, N,N oder O,N steht.

Die so erhaltene Palladium-Verbindung in einer ersten Ausführungsform der Erfindung, liegt in einer Konzentration von 0,01 bis 25 Mol%, bevorzugt von 1 bis 20 Mol%, besonders bevorzugt von 1 bis 15 Mol% und mehr bevorzugt von 1 bis 5 Mol% vor. Die Mol% Angaben beziehen sich jeweils auf das Ausgangsmaterial (a). Bevorzugt ist die Palladium-Verbindung in einer ersten Ausführungsform der Erfindung ein neutraler Palladium-Katalysator. Die Bildung der Palladium-Verbindungen nach den Formeln (I) und/oder (II) kann entweder in situ oder in einem separaten Herstellungsverfahren ex situ erfolgen. Bei einer separaten ex situ Herstellung werden anstatt des Palladium (II)-Salzes und/oder des Palladium(II)-Komplexes (b1) und der Liganden (b2) direkt die Palladium-Verbindungen mit dem Ausgangsmaterial (a) des erfindungsgemäßen Herstellungsverfahrens in Kontakt gebracht.

### Bidentatliganden

Der Einsatz eines Bidentatliganden (b2) ist deshalb vorteilhaft, da dieser zum einen so starke Donoreigenschaften besitzt, dass er das Palladium (II)-Salz und/oder den Palladium(II)-Komplex molekular stabilisieren und somit verhindern kann, dass Palladium(0) ausfällt. Zum anderen kann der Ligand raumgreifende Substituenten besitzen, die eine Chelatisierung von Palladium durch das Ausgangsmaterial (a) verhindern. Wie oben beschrieben, enthält der Ligand N,N-, N,O-, N,S- und/oder O,O-Donoratome. Mehr bevorzugt enthält der Ligand N,N-, N,O- und/oder O,O-Donoratome. Diese Atome können Bestandteil eines zyklischen Systems sein oder über andere geeignete verbrückende Gruppen miteinander verbunden sein.

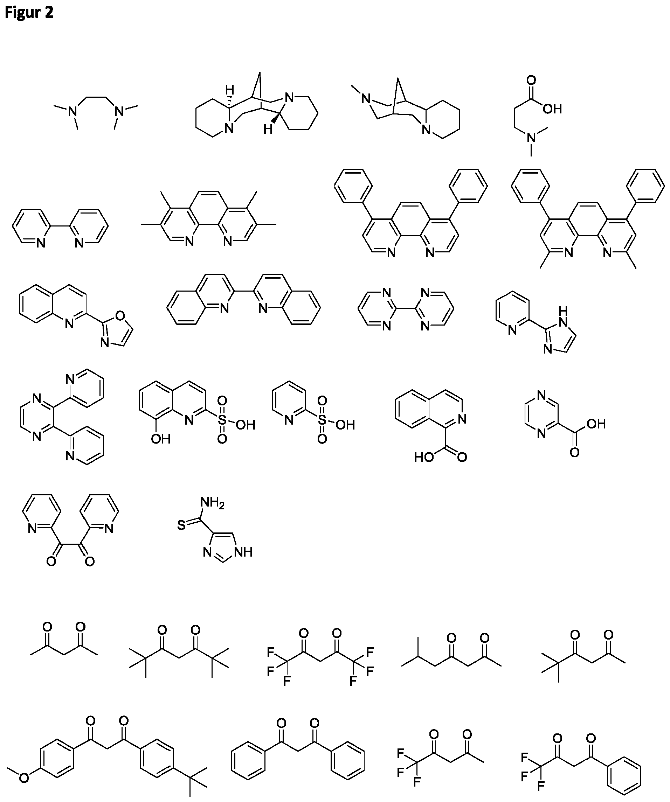

**Fig.2** veranschaulicht Bidentatliganden, die für eine Ausführungsform des erfindungsgemäßen Verfahrens verwendet werden. Diese Abbildung ist beispielhaft und soll keine Einschränkung darstellen.

### Oxidationsmittel

In einer ersten Ausführungsform der Erfindung wird als Oxidationsmittel (b3) ein sauerstoffhaltiges Gas verwendet. Bevorzugt enthält das sauerstoffhaltige Gas Sauerstoff in einer Konzentration von 1 bis 100 Vol.-%, mehr bevorzugt von 5 bis 100 Vol.-% und am meisten bevorzugt von 21 bis 100 Vol.-%.

### Lösungsmittel

In einer ersten Ausführungsform der Erfindung ist das Lösungsmittel (b4) ein polares aprotisches Lösungsmittel. Bevorzugt wird das Lösungsmittel (b4) hierbei aus der Gruppe bestehend aus N,N-disubstituierten offenkettigen und cyclische Säureamiden, wie beispielsweise Dimethylformamid, Dimethylacetamid, Diethylacetamid, Dimethylpropionamid, N-Methylpyrrolidon, aliphatische, cycloaliphatische oder aromatische Nitrile wie Acetonitril, Propionitril oder Benzonitril oder lineare und cyclische Ether, cyclische Lactone und Carbonate. Bevorzugt werden in einer ersten Ausführungsform der Erfindung dem Lösungsmittel (b4) geringe Mengen an Wasser zugesetzt. Bevorzugt in einer Menge von 0,1 bis 25 Vol.-%, mehr bevorzugt in einer Menge von 1-20 Vol.-% und besonders bevorzugt in einer Menge von 5-15 Vol.-%. Die Volumenprozentangaben beziehen sich hierbei auf das Lösungsmittel.

### Verfahrensdurchführung

Weiterhin wird das erfindungsgemäße Verfahren in einer ersten Ausführungsform bei erhöhten Temperaturen durchgeführt. Bevorzugt bei Temperaturen von 50°C bis 120°C, mehr bevorzugt von 60°C bis 100°C, am meisten bevorzugt von 70°C bis 90°C.

In einer zweiten Ausführungsform der Erfindung ist das Lösungsmittel (b4) ein polares aprotisches Lösungsmittel. Bevorzugt wird das Lösungsmittel (b4) hierbei aus der Gruppe bestehend aus N,N-disubstituierten offenkettigen und cyclische Säureamiden, wie beispielsweise Dimethylformamid, Dimethylacetamid, Diethylacetamid, Dimethylpropionamid, N-Methylpyrrolidon, aliphatische, cycloaliphatische oder aromatische Nitrile wie Acetonitril, Propionitril oder Benzonitril oder lineare und cyclische Ether, cyclische Lactone und Carbonate.

Im Kontext der Erfindung kann die Verwendung des Singulars wie "ein" oder "eine" oder "der, die, das" auch den Plural beinhalten, es sei denn aus dem Kontext ergibt sich eindeutig etwas anderes. Beispielsweise der Begriff "das Lösungsmittel" kann auch eine Vielzahl von Lösungsmitteln, einschließlich Mischungen davon, wie beispielsweise eine Lösungsmittelgemisch einschließen.

### Optionale Ausführungsformen

Weiterhin sind in einer weiteren Ausführungsform des erfindungsgemäßen Verfahrens der Co-Katalysator (b5) und die Säure (b6) obligatorisch vorhanden.

Als Co-Katalysatoren (b5) kommen beispielsweise Chinone, Heteropolysäuren und Polyoxometallate oder Metallkomplexe, deren Zentralmetall durch Oxidation mit Sauerstoff leicht zwischen einer Oxidationsstufe II/III, II/IV, IV/V oder I/II wechseln kann, in Frage. Zentralmetalle solcher geeigneten Komplexe können beispielsweise aus der Gruppe Fe, Cu, Mn, Co, Ni, V, ausgewählt werden. Die Co-Katalysatoren können auch in Kombinationen eingesetzt werden, um die Elektronenübergänge während der Redoxprozesse zu unterstützen und zu erleichtern. In einer bevorzugten Ausführungsform werden die Co-Katalysatoren ausgewählt aus der Gruppe bestehend aus Benzochinonen, Napthochinonen, Anthrachinonen, Molybdatophosphorsäure, Molybdatovanadatophosphorsäuren, Wolframatomolybdatophosphorsäuren, Wolframatovanadatophosphorsäuren, Phtalocyanin-Komplexe, FeSO₄, CuCI, CuCl₂, CuSO₄, VOSO₄.

In einer weiteren Ausführungsform der Erfindung können anstatt der Co-Katalysatoren für die Reoxidation von Pd(0) zu Pd(II) auch weitere Oxidationsmittel verwendet werden. Diese sind beispielsweise Peroxide und können aus der Gruppe H₂O₂, t-BuOOH und Metallperoxiden ausgewählt werden.

In einer weiteren Ausführungsform der Erfindung wird der Co-Katalysator (b5) bevorzugt in einer Konzentration von 1 bis 300 Mol% mehr bevorzugt von 5 bis 150 Mol% und am meisten bevorzugt von 21 bis 100 Mol% zugegeben. Die Mol%-Angaben beziehen sich jeweils auf das Ausgangsmaterial (a).

Bevorzugt ist die Säure (b6) in einer weiteren Ausführungsform der Erfindung eine Brönstedsäure und/oder eine Lewis-Säure. Als Brönstedsäuren sind anorganische Mineralsäuren mit schwach koordinierenden Anionen und organische Carbon-, Sulfon- und Phosphonsäuren geeignet. Besonders geeignet sind Sulfonsäuren. Zu den Lewis-Säuren zählen Verbindungen mit unvollständigem oder instabilem Elektronenoktett, wie beispielsweise B(CH₃)₃, BF₃, AlCl₃.

In einer weiteren Ausführungsform der Erfindung wird die Säure (b6) bevorzugt in einer Konzentration von 5 bis 500 Mol%, mehr bevorzugt von 15 bis 300 Mol%, noch mehr bevorzugt von 30 bis 200 Mol% und am meisten bevorzugt von 50 bis 150 Mol% zugegeben. Die Mol%-Angaben beziehen sich jeweils auf das Ausgangsmaterial (a).

In einer weiteren bevorzugten Ausführungsform der Erfindung ist die Säure eine Brönstedsäure. Bevorzugt wird die Brönstedsäure in einer Konzentration von 5 bis 500 Mol%, mehr bevorzugt von 15 bis 300 Mol%, noch mehr bevorzugt von 30 bis 200 Mol% und am meisten bevorzugt von 50 bis 150 Mol% zugegeben. Die Mol%-Angaben beziehen sich jeweils auf das Ausgangsmaterial (a).

Weiterhin wird das Palladium (II)-Salz und/oder der Palladium(II)-Komplex (b1) in einer zweiten Ausführungsform der Erfindung bevorzugt in einer Konzentration von 0,01 bis 20 mol%, mehr bevorzugt von 0,5 bis 10 Mol% und besonders bevorzugt von 1 bis 5 Mol-% verwendet. Die Mol%-Angaben beziehen sich jeweils auf das Ausgangsmaterial (a).

In einer weiteren Ausführungsform der Erfindung wird in einer bevorzugten Weise durch Mischen eines Palladium(II)-Salzes und/oder eines Palladium(II)-Komplexes (b1) mit einer Brönstedsäure (b6) und einem geeigneten Lösungsmittel (b4), vorzugsweise ein polar aprotisches Lösungsmittel, eine Palladium-Verbindung gebildet. Diese Palladium-Verbindung kann entweder in situ oder in einem separaten Herstellungsverfahren ex situ erfolgen. Bei einer separaten ex situ Herstellung werden anstatt des Palladium(II)-Salzes und/oder des Palladium(II)-Komplexes (b1) und der Säure (b6) die präformierten Palladium-Verbindungen mit dem Ausgangsmaterial (a) und dem Co-Katalysator (b5) sowie einem geeigneten Lösungsmittel (b4) des erfindungsgemäßen Herstellungsverfahrens in Kontakt gebracht.

In einer weiteren bevorzugten Ausführungsform ist diese Palladium-Verbindung ein kationischer Palladium-Katalysator.

In einer weiteren Ausführungsform der Erfindung wird die Palladium-Verbindung bevorzugt in einer Konzentration von 0,01 Mol% bis 20 Mol%, bevorzugt in einer Konzentration von 0,5 Mol% bis 10 Mol% und besonders bevorzugt in einer Konzentration von 1 bis 5 Mol% verwendet. Die Mol%-Angaben beziehen sich hierbei jeweils auf das Ausgangsmaterial (a).

Das erfindungsgemäße Verfahren einer weiteren Ausführungsform der Erfindung wird bevorzugt bei Temperaturen zwischen 0°C und 100°C, mehr bevorzugt zwischen 0°C und 50°C, und am meisten bevorzugt zwischen 0°C und 25°C durchgeführt.

Die durch das erfindungsgemäße Herstellungsverfahren gewonnenen ungesättigten makrozyklischen Monoketonen können durch übliche Trennverfahren, beispielweise durch präparative Hochleistungsflüssigkeitschromatographie (HPLC) oder fraktionierte Destillation aufgereinigt werden.

### GEWERBLICHE ANWENDBARKEIT

Gemäß der vorliegenden Erfindung können ungesättigte makrozyklische Monoketone hergestellt werden, von makrozyklischen Dienen mit einer Ringgröße von mindestens 9 Kohlenstoffatomen; und bevorzugt von makrozyklischen Dienen mit einer Ringgröße von 16 Kohlenstoffatomen, die beispielsweise zu wertvollen Duft-, Aroma- oder Geschmacksstoffen weiter verarbeitet werden können.

### BEISPIELE

### Beispiele 1 bis 8

1,9-CHDD (Isomerengemisch; 0.120 g, 0.55 mmol) in 2.5 mL N,N-Dimethylacetamid und 0.2 mL H₂O wurde in den Glaseinsatz eines Autoklaven gegeben und die berechnete Menge des Pd(II)-Katalysators wurde hinzugefügt (0.01-20 Mol%). Der Autoklav wurde mit Argon und Sauerstoff gespült und dann wurde der entsprechende Sauerstoffdruck aufgepresst (3 bar). Danach wurde die Reaktionsmischung in einem Autoklaven bei 90 °C±1 °C für 15 h gerührt. Nach Beendigung der Reaktion wurde der Autoklav abgekühlt und entspannt. Das Reaktionsgemisch wurde mittels Zugabe von THF (Tetrahydrofuran) homogenisiert. GC/MS wurde für die qualitative Analyse der Produkte und die GC-FID-Methode zusammen mit einem internen Standard für die Quantifizierung der Produkte genutzt. Die Ergebnisse sind in **Tabelle 1** dargestellt. X steht hierbei für den Umsatz, S für die Selektivität und Y für die Ausbeute.

**Tab.1**

| Ergebnisse Beispiele 1 bis 8 | | | | | |
|---|---|---|---|---|---|
| **Beispiel** | **Katalysator** | **Katalysator [Mol%]** | **X_{1,9-CHDD} [Mol%]** | **S_{8-CHD}¹ [Mol%]** | **S_{KETONES}¹ [Mol%]** |
| 1 | Bis(2,4-pentanedionato)-palladium(II) | 1 | 16 | 39 | 88 |
| | | 3 | 20 | 39 | 86 |
| | | 5 | 27 | 35 | 69 |
| | | 10 | 38 | 33 | 69 |
| | | 20 | 43 | 35 | 69 |
| 2 | Bis(2,2,6,6-tetramethyl-3,5-heptanedionato)palladium(II) | 1 | 12 | 39 | 80 |
| | | 3 | 18 | 42 | 84 |
| | | 5 | 26 | 43 | 84 |
| | | 10 | 40 | 35 | 67 |
| | | 20 | 49 | 42 | 66 |
| 3 | Bis(5,5-dimethyl-2,4-hexanedionato)palladium(II) | 1 | 8 | 50 | 99 |
| | | 3 | 15 | 45 | 84 |
| | | 5 | 16 | 52 | 94 |
| 4 | Bis(6-methyl-2,4-heptanedionato)palladium(II) | 1 | 14 | 35 | 83 |
| | | 3 | 30 | 29 | 69 |
| | | 5 | 45 | 26 | 64 |
| | | 10 | 56 | 24 | 56 |
| 5 | Bis(1,3-diphenylpropane-1,3-dionato)palladium(II) | 1 | 27 | 31 | 74 |
| | | 3 | 43 | 33 | 72 |
| | | 5 | 46 | 31 | 67 |
| 6 | Bis(1-(4-methoxyphenyl)-3-(4-tert-butylphenyl)propane-1,3-dionate)palladium(II) | 1 | 13 | 39 | 95 |
| | | 3 | 31 | 37 | 80 |
| | | 5 | 32 | 39 | 81 |
| 7 | Bis(1,1,1-trifluoro-2,4-pentanedionato)palladium(II) | 0.1 | 15 | 22 | 70 |
| | | 0.5 | 27 | 21 | 68 |
| | | 1 | 54 | 18 | 59 |
| | | 3 | 77 | 14 | 42 |
| | | 5 | 76 | 14 | 38 |
| | | 10 | 76 | 20 | 41 |
| 8 | Bis(1,1,1,5,5,5-hexafluoro-2,4-pentanedionato)palladium(II) | 0.01 | 10 | 28 | 66 |
| | | 0.05 | 18 | 20 | 65 |
| | | 0.1 | 23 | 21 | 65 |
| | | 0.5 | 48 | 19 | 60 |
| | | 1 | 71 | 13 | 45 |
| | | 3 | 73 | 11 | 37 |
| | | 5 | 73 | 12 | 32 |
| | | 10 | 51 | 13 | 40 |

| | | | | | |
|---|---|---|---|---|---|
| ¹ bezogen auf **X_{1,9-CHDD}** | | | | | |

### Beispiele 9 bis 13

### Bis(2,4-pentanedionato)palladium(II) und verschiedene Lösungsmittel

1,9-CHDD (Isomerengemisch; 0.120 g, 0.55 mmol) in 2.5 mL des Lösungsmittels (N,N-Dimethylformamid, N,N-Dimethylacetamid, N,N-Dimethylpropionamid oder N-methyl-2-pyrrolidon) und 0.2 mL H₂O wurde in den Glaseinsatz eines Autoklaven gegeben und Bis(2,4-pentanedionato)palladium(II) (3 Mol%) wurde hinzugefügt. Der Autoklav wurde mit Argon und Sauerstoff gespült und dann wurde der entsprechende Sauerstoffdruck aufgepresst (3 bar). Danach wurde die Reaktionsmischung in einem Autoklaven bei 90 °C±1 °C für 15 h gerührt. Nach Beendigung der Reaktion wurde der Autoklav abgekühlt und entspannt. Das Reaktionsgemisch wurde mittels Zugabe von THF homogenisiert. GC/MS wurde für die qualitative Analysise der Produkte und die GC-FID-Methode zusammen mit einem internen Standard für die Quantifizierung der Produkte genutzt. Die Ergebnisse sind in **Tabelle 2** dargestellt.

**Tab. 2**

| Ergebnisse Beispiel 9 bis 13 | | | | | |
|---|---|---|---|---|---|
| **Beispiel** | **Lösungsmittel** | **Zeit [h]** | **X_{1,9-CHDD} [Mol%]** | **S_{8-CHD}¹ [Mol%]** | **S_{KETONES}¹ [Mol%]** |
| 9 | *N,N*-Dimethylformamid | 15 | 0 | 0 | 0 |
| 10 | *N,N*-Dimethylacetamid | 15 | 20 | 39 | 86 |
| 11 | *N*,*N*-Dimethylpropionamid | 15 | 9 | 17 | 33 |
| 12 | *N*-Methyl-2-pyrrolidon | 15 | 61 | 22 | 59 |
| 13 | *N*-Methyl-2-pyrrolidon | 5 | 11 | 39 | 94 |

| | | | | | |
|---|---|---|---|---|---|
| ¹ bezogen auf **X_{1,9-CHDD}** | | | | | |

### Beispiele 14 bis 16

### Bis(2,4-pentanedionato)palladium(II) und Co-Katalysatoren

1,9-CHDD (Isomerengemisch; 0.120 g, 0.55 mmol) in 2.5 mL N,N-Dimethylacetamid und 0.2 mL H₂O wurde in den Glaseinsatz eines Autoklaven gegeben und Bis(2,4-pentane-dionato)palladium(II) (1 Mol%) sowie die betreffende Menge eines Co-Katalysators wurde hinzugefügt. Der Autoklav wurde mit Argon und Sauerstoff gespült und dann wurde der entsprechende Sauerstoffdruck aufgepresst (3 bar). Danach wurde die Reaktionsmischung im Autoklaven bei 90 °C±1 °C für 15 h gerührt. Nach Beendigung der Reaktion wurde der Autoklav abgekühlt und entspannt. Das Reaktionsgemisch wurde mittels Zugabe von THF homogenisiert. GC/MS wurde für die qualitative Analyse der Produkte und die GC-FID-Methode zusammen mit einem internen Standard für die Quantifizierung der Produkte genutzt. Die Ergebnisse sind in **Tabelle 3** dargestellt.

**Tab. 3**

| Ergebnisse Bespiele 14 bis 16 | | | | | |
|---|---|---|---|---|---|
| **Beispiel** | **Co-Katalysator** | **Co-Kat. [Mol%]** | **X_{1,9-CHDD} [Mol%]** | **S_{8-CHD}¹ [Mol%]** | **S_{KETONE}¹ [Mol%]** |
| 14 | CuCl₂ | 2 | 0 | 0 | 0 |
| 15 | Cu(acetylacetonat)₂ | 2 | 11 | 27 | 80 |
| 16 | Benzochinon | 100 | 0 | 0 | 0 |

| | | | | | |
|---|---|---|---|---|---|
| ¹ bezogen auf **X_{1,9-CHDD}** | | | | | |

### Beispiele 17 bis 27 (Referenzbeispiele)

### Pd(OAc)₂ bzw. Pd(NO₃)₂ · 2 H₂O und verschiedene Lösungsmittelverhältnisse

In einem verschließbarem 4-ml-Glasreaktor mit Schraubkappe und Septum wird zu einem Lösungsmittelsystem, bestehend aus N,N-Dimethylacetamid, Acetonitril und Wasser (Vges. = 3,06 ml), 1,9-CHDD (Isomerengemisch; 44 mg; 0,2 mmol) vorgelegt. Zu dieser Lösung werden der Palladium(II)-Präkursor (0,01 mmol; 5,0 Mol%); Benzochinon (22 mg; 0,2 mmol) und eine wässrige Tetrafluorborsäure (50 Gew%; 125 µl; 0,83 mmol) gegeben. Der Reaktor wird verschlossen, das Septum mit einer Einwegnadel durchstochen und die Reaktionslösung für 20 Stunden bei Raumtemperatur intensiv gerührt. Im Anschluss wird die Reaktionslösung mithilfe von Tetrahydrofuran auf ein konstantes Volumen aufgefüllt und homogenisiert. Die qualitative und quantitative Analyse der Reaktionsprodukte erfolgt anhand eines internen Standards (n-Hexadecan) an einem GC/MS mit FID. Die Ergebnisse sind in **Tabelle 4** dargestellt.

**Tab. 4**

| Ergebnisse Beispiele 17 bis 27 | | | | | | |
|---|---|---|---|---|---|---|
| **Beispiel** | **Palladium(II)-Präkursor** | **LM-Verhältnis DMA/MeCN/H₂O [Vol%]** | **X_{1,9-CHDD} [Mol%]** | **S_{8-CHD}¹ [Mol%]** | **S_{DIKETON}¹ [Mol%]** | **Y_{8-CHD}¹ [Mol%]** |
| 17 | Palladium(II)-acetat | 0/7/1 | 26 | 53 | 5 | 14 |
| 18 | Palladium(II)-acetat | 1/6/1 | 26 | 73 | 8 | 19 |
| 19 | Palladium(II)-acetat | 2/5/1 | 32 | 76 | 8 | 25 |
| 20 | Palladium(II)-acetat | 3/4/1 | 39 | 67 | 11 | 26 |
| 21 | Palladium(II)-acetat | 4/3/1 | 40 | 72 | 9 | 29 |
| 22 | Palladium(II)-acetat | 5/2/1 | 46 | 67 | 12 | 31 |
| 23 | Palladium(II)-acetat | 6/1/1 | 44 | 63 | 7 | 28 |
| 24 | Palladium(II)-nitrat Dihydrat | 4/3/0 | 31 | 84 | 2 | 26 |
| 25 | Palladium(II)-nitrat Dihydrat | 8/6/1 | 42 | 81 | 5 | 34 |
| 26 | Palladium(II)-nitrat Dihydrat | 4/3/1 | 45 | 79 | 8 | 35 |
| 27 | Palladium(II)-nitrat Dihydrat | 4/3/2 | 34 | 62 | 13 | 21 |

| | | | | | | |
|---|---|---|---|---|---|---|
| ¹ bezogen auf **X_{1,9-CHDD}** | | | | | | |

### Beispiele 28 bis 34 (Referenzbeispiele)

### Dimethylacetamid (DMA)/MeCN/H₂O = 4/3/1 und Variation des Palladium (II)-Präkursors

In einem verschließbarem 4-ml-Glasreaktor mit Schraubkappe und Septum wird zu einem Lösungsmittelsystem, bestehend aus N,N-Dimethylacetamid, Acetonitril und Wasser (DMA/MeCN/H2O = 4/3/1; Vges. = 3,06 ml), 1,9-CHDD (Isomerengemisch; 44 mg; 0,2 mmol) vorgelegt. Zu dieser Lösung werden der Palladium(II)-Präkursor (0,01 mmol; 5,0 Mol%); Benzochinon (22 mg; 0,2 mmol) und eine wässrige Tetrafluorborsäure (50 Gew%; 125 µl; 0,83 mmol) gegeben. Der Reaktor wird verschlossen, das Septum mit einer Einwegnadel durchstochen und die Reaktionslösung für 20 Stunden bei Raumtemperatur intensiv gerührt. Im Anschluss wird die Reaktionslösung mithilfe von Tetrahydrofuran auf ein konstantes Volumen aufgefüllt und homogenisiert. Die qualitative und quantitative Analyse der Reaktionsprodukte erfolgt anhand eines internen Standards (n-Hexadecan) an einem GC/MS mit FID. Die Ergebnisse sind in **Tabelle 5** dargestellt.

**Tab. 5**

| Ergebnisse Beispiel 28 bis 34 | | | | | |
|---|---|---|---|---|---|
| **Beispiel** | **Palladium(II)-Präkursor** | **X_{1,9-CHDD} [Mol%]** | **S_{8-CHD}¹ [Mol%]** | **S_{DIKETON}¹ [Mol%]** | **Y_{8-CHD}¹ [Mol%]** |
| 28 | Palladium(II)-sulfat Dihydrat | 41 | 76 | 6 | 31 |
| 29 | Bis(2,2,6,6-tetramethyl-3,5-heptanedionato) palladium(II) | 9 | 75 | 0 | 7 |
| 30 | Palladium(II)-nitrat Dihydrat | 45 | 79 | 8 | 35 |
| 31 | Bis(triphenylphosphin) palladium(II)-dichlorid | 0 | 0 | 0 | 0 |
| 32 | Bis(acetonitril) dichloropalladium(II) | 3 | 87 | 0 | 3 |
| 33 | Palladium(II)-chlorid | 5 | 64 | 0 | 3 |
| 34 | Palladium(II)-acetat | 43 | 72 | 8 | 31 |

| | | | | | |
|---|---|---|---|---|---|
| ¹ bezogen auf **X_{1,9-CHDD}** | | | | | |

### Beispiele 35 bis 41 (Referenzbeispiele)

### DMA/MeCN/H₂O = 4/3/1 und Variation der Pd(NO₃)₂ · 2 H₂O -Katalysatormenge

In einem verschließbarem 4-ml-Glasreaktor mit Schraubkappe und Septum wird zu einem Lösungsmittelsystem, bestehend aus N,N-Dimethylacetamid, Acetonitril und Wasser (DMA/MeCN/H₂O = 4/3/1; Vges. = 3,06 ml), 1,9-CHDD (Isomerengemisch; 44 mg; 0,2 mmol) vorgelegt. Zu dieser Lösung werden Palladium(II)-nitrat Dihydrat (0,1-20 Mol%); Benzochinon (22 mg; 0,2 mmol) und eine wässrige Tetrafluorborsäure (50 Gew%; 125 µl; 0,83 mmol) gegeben. Der Reaktor wird verschlossen, das Septum mit einer Einwegnadel durchstochen und die Reaktionslösung für 20 Stunden bei Raumtemperatur intensiv gerührt. Im Anschluss wird die Reaktionslösung mithilfe von Tetrahydrofuran auf ein konstantes Volumen aufgefüllt und homogenisiert. Die qualitative und quantitative Analyse der Reaktionsprodukte erfolgt anhand eines internen Standards (n-Hexadecan) an einem GC/MS mit FID. Die Ergebnisse sind in **Tabelle 6** dargestellt.

**Tab. 6**

| Ergebnisse Beispiel 35 bis 41 | | | | | |
|---|---|---|---|---|---|
| **Beispiel** | **Pd(NO₃)₂-Menge [Mol%]** | **X_{1,9-CHDD} [Mol%]** | **S_{8-CHD}¹ [Mol%]** | **S_{DIKETON}¹ [Mol%]** | **Y_{8-CHD}¹ [Mol%]** |
| 35 | 0,1 | 3 | 15 | 0 | 0 |
| 36 | 0,5 | 7 | 51 | 0 | 3 |
| 37 | 1,0 | 10 | 67 | 0 | 7 |
| 38 | 2,5 | 21 | 81 | 2 | 17 |
| 39 | 5,0 | 45 | 79 | 8 | 35 |
| 40 | 10,0 | 42 | 64 | 6 | 27 |
| 41 | 20,0 | 52 | 51 | 5 | 27 |

| | | | | | |
|---|---|---|---|---|---|
| ¹ bezogen auf **X_{1,9-CHDD}** | | | | | |

### Beispiele 42 bis 48 (Referenzbeispiele)

### DMA/MeCN/H₂O = 4/3/1; Pd(NO₃)₂ · 2 H₂O und Variation der Reaktionstemperatur

In einem 25 ml Rundkolben wird zu einem Lösungsmittelsystem, bestehend aus N,N-Dimethylacetamid, Acetonitril und Wasser (DMA/MeCN/H₂O = 4/3/1; Vges. = 15,3 ml), 1,9-CHDD (Isomerengemisch; 220 mg; 1 mmol) vorgelegt. Zu dieser Lösung werden Palladium (II)-nitrat Dihydrat (0,05 mmol; 5,0 Mol%); Benzochinon (108 mg; 1 mmol) und eine wässrige Tetrafluorborsäure (50 Gew%; 625 µl; 4,15 mmol) gegeben. Der Reaktor wird mit einem Stopfen verschlossen und die Reaktionslösung für 20 Stunden in einem Temperaturbereich von 0-80 °C intensiv gerührt. Im Anschluss wird der Reaktor langsam auf Raumtemperatur erwärmt/abgekühlt und die Reaktionslösung mithilfe von Tetrahydrofuran auf ein konstantes Volumen aufgefüllt und homogenisiert. Die qualitative und quantitative Analyse der Reaktionsprodukte erfolgt anhand eines internen Standards (n-Hexadecan) an einem GC/MS mit FID. Die Ergebnisse sind in **Tabelle 7** dargestellt.

**Tab. 7**

| Ergebnisse Beispiel 42 bis 48 | | | | | |
|---|---|---|---|---|---|
| **Beispiel** | **Temperatur [°C]** | **X_{1,9-CHDD} [Mol%]** | **S_{8-CHD}¹ [Mol%]** | **S_{DIKETON}¹ [Mol%]** | **Y_{8-CHD}¹ [Mol%]** |
| 42 | 0 | 29 | 88 | 3 | 25 |
| 43 | 25 | 39 | 79 | 8 | 31 |
| 44 | 40 | 37 | 82 | 5 | 30 |
| 45 | 50 | 34 | 81 | 3 | 27 |
| 46 | 60 | 32 | 82 | 3 | 26 |
| 47 | 70 | 32 | 76 | 2 | 25 |
| 48 | 80 | 31 | 67 | 2 | 21 |

| | | | | | |
|---|---|---|---|---|---|
| ¹ bezogen auf **X_{1,9-CHDD}** | | | | | |

### Beispiele 49 bis 54 (Referenzbeispiele)

### DMA/MeCN/H₂O = 8/6/1; Pd(NO₃)₂ ·2 H₂O und Variation der Brönsted-Säure bzw. eines Säureanions

In einem verschließbarem 4-ml-Glasreaktor mit Schraubkappe und Septum wird zu einem Lösungsmittelsystem, bestehend aus N,N-Dimethylacetamid, Acetonitril und Wasser (DMA/MeCN/H₂O = 8/6/1; Vges. = 3,06 ml), 1,9-CHDD (Isomerengemisch; 44 mg; 0,2 mmol) vorgelegt. Zu dieser Lösung werden Palladium(II)-nitrat Dihydrat (0,01 mmol; 5,0 Mol%); Benzochinon (22 mg; 0,2 mmol) und eine Säure bzw- ein Säureanion in Form eines Silbersalzes (0,83 mmol) gegeben. Der Reaktor wird verschlossen, das Septum mit einer Einwegnadel durchstochen und die Reaktionslösung für 20 Stunden bei Raumtemperatur intensiv gerührt. Im Anschluss wird die Reaktionslösung mithilfe von Tetrahydrofuran auf ein konstantes Volumen aufgefüllt und homogenisiert. Die qualitative und quantitative Analyse der Reaktionsprodukte erfolgt anhand eines internen Standards (n-Hexadecan) an einem GC/MS mit FID. Die Ergebnisse sind in **Tabelle 8** dargestellt.

**Tab. 8:**

| Ergebnisse Beispiel 49 bis 54 | | | | | |
|---|---|---|---|---|---|
| **Beispiel** | **Säure/Silbersalz** | **X_{1.9-CHDD} [Mol%]** | **S_{8-CHD}¹ [Mol%]** | **S_{DIKETON}¹ [Mol%]** | **Y_{8-CHD}¹ [Mol%]** |
| 49 | Tetrafluorborsäure | 42 | 81 | 5 | 34 |
| 50 | Methansulfonsäure | 51 | 75 | 9 | 38 |
| 51 | Perchlorsäure | 49 | 67 | 4 | 33 |
| 52 | para-Toluolsulfonsäure | 54 | 76 | 7 | 41 |
| 53 | Silbertrifluormethan-sulfonat | 17 | 54 | 0 | 9 |
| 54 | Aquivion^{®} | 1 | 0 | 0 | 0 |

| | | | | | |
|---|---|---|---|---|---|
| ¹ bezogen auf **X_{1,9-CHDD}** | | | | | |

### Beispiele 55 bis 63 (Referenzbeispiele)

### DMA/MeCN/H₂O = 8/6/1; Pd(NO₃)₂ · 2 H₂O und Variation der p-TsOH-Konzentration

In einem verschließbarem 4-ml-Glasreaktor mit Schraubkappe und Septum wird zu einem Lösungsmittelsystem, bestehend aus N,N-Dimethylacetamid, Acetonitril und Wasser (DMA/MeCN/H₂O = 8/6/1; Vges. = 3,06 ml), 1,9-CHDD (Isomerengemisch; 44 mg; 0,2 mmol) vorgelegt. Zu dieser Lösung werden Palladium (II)-nitrat Dihydrat (0,01 mmol; 5,0 Mol%); Benzochinon (22 mg; 0,2 mmol) und para-Toluolsulfonsäure Monohydrat (5-430 Mol%) gegeben. Der Reaktor wird verschlossen, das Septum mit einer Einwegnadel durchstochen und die Reaktionslösung für 20 Stunden bei Raumtemperatur intensiv gerührt. Im Anschluss wird die Reaktionslösung mithilfe von Tetrahydrofuran auf ein konstantes Volumen aufgefüllt und homogenisiert. Die qualitative und quantitative Analyse der Reaktionsprodukte erfolgt anhand eines internen Standards (n-Hexadecan) an einem GC/MS mit FID. Die Ergebnisse sind in Tabelle 9 dargestellt.

**Tab. 9**

| Ergebnisse Beispiel 55 bis 63 | | | | | |
|---|---|---|---|---|---|
| **Beispiel** | **Säurekonzentration [Mol%]** | **X_{1,9-CHDD} [Mol%]** | **S_{8-CHD}¹ [Mol%]** | **S_{DIKETON}¹ [Mol%]** | **Y_{8-CHD}¹ [Mol%]** |
| 55 | 5 | 18 | 60 | 0 | 11 |
| 56 | 12,5 | 22 | 66 | 0 | 15 |
| 57 | 50 | 41 | 74 | 4 | 30 |
| 58 | 100 | 45 | 75 | 5 | 34 |
| 59 | 125 | 47 | 79 | 5 | 37 |
| 60 | 150 | 47 | 74 | 5 | 35 |
| 61 | 175 | 52 | 74 | 6 | 39 |
| 62 | 200 | 54 | 76 | 8 | 41 |
| 63 | 415 | 53 | 78 | 7 | 41 |

| | | | | | |
|---|---|---|---|---|---|
| ¹ bezogen auf **X_{1,9-CHDD}** | | | | | |

### Beispiele 64 bis 69 (Referenzbeispiele)

### DMA/MeCN/H₂O = 8/6/1; 125 Mol-% p-TsOH und Variation der Pd(NO₃)₂ · 2 H₂O - Katalysatormenge

In einem verschließbarem 4-ml-Glasreaktor mit Schraubkappe und Septum wird zu einem Lösungsmittelsystem, bestehend aus N,N-Dimethylacetamid, Acetonitril und Wasser (DMA/MeCN/H₂O = 8/6/1; Vges. = 3,06 ml), 1,9-CHDD (Isomerengemisch; 44 mg; 0,2 mmol) vorgelegt. Zu dieser Lösung werden Palladium (II)-nitrat Dihydrat (0,1-10 Mol%); Benzochinon (22 mg; 0,2 mmol) und para-Toluolsulfonsäure Monohydrat (48 mg; 0,25 mmol) gegeben. Der Reaktor wird verschlossen, das Septum mit einer Einwegnadel durchstochen und die Reaktionslösung für 20 Stunden bei Raumtemperatur intensiv gerührt. Im Anschluss wird die Reaktionslösung mithilfe von Tetrahydrofuran auf ein konstantes Volumen aufgefüllt und homogenisiert. Die qualitative und quantitative Analyse der Reaktionsprodukte erfolgt anhand eines internen Standards (n-Hexadecan) an einem GC/MS mit FID. Die Ergebnisse sind in **Tabelle 10** dargestellt.

**Tab. 10**

| Ergebnisse Beispiel 64 bis 69 | | | | | |
|---|---|---|---|---|---|
| **Beispiel** | **Pd(NO₃)₂-Menge [Mol%]** | **X_{1,9-CHDD} [Mol%]** | **S_{8-CHD}¹ [Mol%]** | **S_{DIKETON}¹ [Mol%]** | **Y_{8-CHD}¹ [Mol%]** |
| 64 | 0,1 | 2 | 42 | 0 | 1 |
| 65 | 0,5 | 6 | 75 | 0 | 5 |
| 66 | 1,0 | 12 | 76 | 0 | 10 |
| 67 | 2,5 | 27 | 81 | 2 | 22 |
| 68 | 5,0 | 47 | 79 | 5 | 37 |
| 69 | 10,0 | 68 | 68 | 11 | 46 |

| | | | | | |
|---|---|---|---|---|---|
| ¹ bezogen auf **X_{1,9-CHDD}** | | | | | |

## Patentansprüche

1. Verfahren zur Herstellung von ungesättigten makrozyklischen Monoketonen umfassend die folgenden Schritte
(a) Bereitstellung von makrozyklischen Dienen mit einer Ringgröße von mindestens 9 Kohlenstoffatomen;
(b) Inkontaktbringen der Ausgangsstoffe aus Schritt (a) mit
(b1) einem Palladium(II)-Salz und/oder einem Palladium(II)-Komplex; sowie
(b2) einem Bidentatligand, der N,N-, N,O-, N,S- oder O,O-Donoratome enthält, und
(b3) einem Oxidationsmittel; und
(b4) einem Lösungsmittel; und gegebenenfalls
(b5) einem Co-Katalysator; und gegebenenfalls
(b6) einer Säure.

2. Verfahren nach Anspruch 1, **dadurch gekennzeichnet, dass** das Lösungsmittel (b4) ein polares aprotisches Lösungsmittel ist und bevorzugt ausgewählt ist aus der Gruppe bestehend aus N,N-disubstituierten offenkettigen und cyclische Säureamiden, wie beispielsweise Dimethylformamid, Dimethylacetamid, Diethylacetamid, Dimethylpropionamid, N-Methylpyrrolidon, aliphatische, cycloaliphatische oder aromatische Nitrile wie Acetonitril, Propionitril oder Benzonitril oder lineare und cyclische Ether, cyclische Carbonate und Lactone.

3. Verfahren nach Anspruch 1 und/oder 2, **dadurch gekennzeichnet, dass** das Palladium(II)-Salz und/oder der Palladium(II)-Komplex (b1) ausgewählt sind aus der Gruppe bestehend aus Palladiumchlorid, Palladiumbromid, H₂PdCl4, Li₂PdCl₄, Na₂PdCl₄, K₂PdCl₄, (NH₄)₂PdCl₄, Palladiumacetat, Palladiumtrifluoroacetat, Palladiumbenzoat, Palladiumnitrat, Palladiumsulfat, Tetrakis(acetonitril)palladium(II) tetrafluoroborat und/oder Tetrakis(acetonitril)palladium(II) bis(trifluoromethanesulfonat).

4. Verfahren nach einem der Ansprüche 1 bis 3, **dadurch gekennzeichnet, dass** das Palladium(II)-Salz und/oder der Palladium(II)-Komplex (b1) mit dem Bidentatligand (b2) eine Palladium-Verbindung der Formel (I) und/oder der Formel (II) bilden 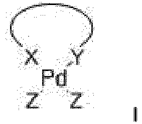
- wobei X und Y jeweils unabhängig voneinander für N,N, O,N oder O,O steht; und
- wobei Z jeweils unabhängig voneinaner für ein Halogen, Acetat, Trimethylacetat, Trifluormethylacetat, MeCN, PhCN, NO₂, NO, Nitrat, Nitrit oder Sulfat steht; 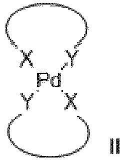
- wobei X und Y jeweils unabhängig voneinander für O,O, N,N oder O,N steht.

5. Verfahren nach Anspruch 4, **dadurch gekennzeichnet, dass** die Palladium-Verbindung in einer Konzentration von 0,01 bis 25 Mol%, bevorzugt von 1 bis 20 Mol%, besonders bevorzugt von 1 bis 5 Mol%, jeweils bezogen auf das Ausgangsmaterial (a), zugegeben wird.

6. Verfahren nach einem der Ansprüche 1 bis 5, **dadurch gekennzeichnet, dass** als Oxidationsmittel (b3) ein sauerstoffhaltiges Gas verwendet wird.

7. Verfahren nach Anspruch 6, **dadurch gekennzeichnet, dass** das sauerstoffhaltige Gas Sauerstoff in einer Konzentration von 1 bis 100 Vol.-%, bevorzugt von 5 bis 100 Vol.-% und am meisten bevorzugt von 21 bis 100 Vol.-% enthält.

8. Verfahren nach einem der Ansprüche 1 bis 7, **dadurch gekennzeichnet, dass** das Verfahren bei Temperaturen von 50°C bis 120°C, bevorzugt von 60°C bis 100°C, am meisten bevorzugt von 70°C bis 90°C durchgeführt wird.

9. Verfahren nach Anspruch 1, **dadurch gekennzeichnet, dass** der Co-Katalysator (b5) und die Säure (b6) obligatorisch vorhanden sind.

10. Verfahren nach Anspruch 9, **dadurch gekennzeichnet, dass**
(i) der Co-Katalysator (b5) ausgewählt ist aus der Gruppe bestehend aus Benzochinonen, Naphthochinonen, Anthrachinonen, Molybdatophosphorsäure, Molybdatovanadatophosphorsäuren, Wolframatomolybdatophosphorsäuren, Wolframatovanadatophosphorsäuren, Phthalocyanin-Komplexe, FeSO₄, CuCI, CuCl₂, CuSO₄, VOSO₄ und
(ii) die Säure (b6) eine Brönstedsäure oder Lewis-Säure ist.

11. Verfahren nach Anspruch 10, **dadurch gekennzeichnet, dass** der Co-Katalysator (b5) in einer Konzentration von 1 bis 300 Mol%, bevorzugt von 5 bis 150 Mol% und am meisten bevorzugt von 21 bis 100 Mol%, jeweils bezogen auf das Ausgangsmaterial (a), zugegeben wird.

12. Verfahren nach Anspruch 10, **dadurch gekennzeichnet, dass** die Säure (b6) in einer Konzentration von 5 bis 500 Mol%, bevorzugt von 15 bis 300 Mol%, mehr bevorzugt von 30 bis 200 Mol% und am meisten bevorzugt von 50 bis 150 Mol%, jeweils bezogen auf das Ausgangsmaterial (a), zugegeben wird.

13. Verfahren nach Anspruch 9, **dadurch gekennzeichnet, dass** das Palladium(II)-Salz und/oder der Palladium(II)-Komplex (b1) in einer Konzentration von 0,01 bis 20 Mol%, bevorzugt von 0,5 bis 10 Mol% und besonders bevorzugt von 1 bis 5 Mol%, jeweils bezogen auf das Ausgangsmaterial (a), zugegeben wird.

14. Verfahren nach Anspruch 10, **dadurch gekennzeichnet, dass** das Verfahren bei Temperaturen zwischen 0°C und 100°C, bevorzugt zwischen 0°C und 50°C, und am meisten bevorzugt zwischen 0°C und 25°C durchgeführt wird.

## Claims

1. A process for the preparation of unsaturated macrocyclic monoketones comprising the following steps
(a) providing macrocyclic dienes having a ring size of at least 9 carbon atoms;
(b) contacting the starting materials of step (a) with
(b1) a palladium (II) salt and/or a palladium (II) complex; and
(b2) a bidentate ligand containing N,N-, N,O-, N,S- or O,O-donor atoms, and
(b3) an oxidizing agent; and(b4) a solvent; and optionally
(b5) a co-catalyst; and optionally
(b6) an acid.

2. The process according to claim 1, **characterized in that** the solvent (b4) is a polar aprotic solvent and is preferably selected from the group consisting of N,N-disubstituted open chain and cyclic acid amides, such as dimethylformamide,dimethylacetamide, diethylacetamide, dimethylpropio-namide, N-methylpyrrolidone, aliphatic, cycloaliphatic or aromatic nitriles such as acetonitrile, propionitrile or benzonitrile or linear and cyclic ethers, cyclic car-bonates and lactones.

3. The process according to claim 1 and/or 2, **characterized in that** the palladium (II) salt and/or the palladium (II) complex (b1) are selected from the group consisting of palladium chloride, palladium bromide, H₂PdCl₄, Li₂PdCl₄, Na₂PdCl₄, K₂PdCl₄, (NH₄)₂PdCl₄, palladium acetate, palladium trifluoroacetate, palladium benzoate, palladium nitrate, palladium sulfate, tetrakis(acetonitrile)palladium (II) tetrafluoroborate and/or tetra-kis(acetonitrile)palladium (II) bis(trifluoromethanesulfonate).

4. The process according to any one of claims 1 to 3, wherein the palladium (II) salt and/or the palladium (II) complex (b1) with the bidentate ligand (b2) form a palladium compound of the formula (I) and/or of the formula (II) 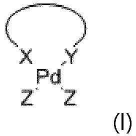
- wherein X and Y are each independently N,N, O,N or O,O; and
- wherein Z is each independently a halogen, acetate, trimethyl acetate, trifluoromethyl acetate, MeCN, PhCN, NO₂, NO, nitrate, nitrite or sulfate; 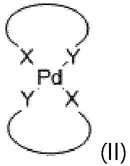
- wherein X and Y each independently represent O,O, N,N or O,N.

5. The process according to claim 4, **characterized in that** the palladium compound is added in a concentration of from 0.01 to 25 mol%, preferably from 1 to 20 mol%, particularly preferably from 1 to 5 mol%, in each case based on the starting material (a).

6. The process according to any one of claims 1 to 5, **characterized in that** an oxygen-containing gas is used as oxidizing agent (b3).

7. The process according to claim 6, **characterized in that** the oxygen-containing gas contains oxygen in a concentration of from 1 to 100% by volume, preferably from 5 to 100% by volume and most preferably from 21 to 100% by volume.

8. The process according to any one of claims 1 to 7, **characterized in that** the process is carried out at temperatures from 50°C to 120°C, preferably from 60°C to 100°C, most preferably from 70°C to 90°C.

9. The process according to claim 1, **characterized in that** the co-catalyst (b5) and the acid (b6) are obligatorily present.

10. The process according to claim 9, **characterized in that**
(i) the co-catalyst (b5) is selected from the group consisting of benzoquinones, naphthoquinones, anthraquinones, molybdophosphoric acid, molybdatovanadatophosphoric acids, tungstomolybdophosphoric acids, tungstomato-vanadato phosphoric acids, phthalocyanine complexes, FeSO₄, CuCI, CuClz, CuSO₄, VOSO₄, and
(ii) the acid (b6) is a Brönsted acid or Lewis acid.

11. The process according to claim 10, **characterized in that** the co-catalyst (b5) is added in a concentration of from 1 to 300 mol%, preferably from 5 to 150 mol% and most preferably from 21 to 100 mol%, in each case based on the starting material (a).

12. The process according to claim 10, **characterized in that** the acid (b6) is added in a concentration of from 5 to 500 mol%, preferably from 15 to 300 mol%, more preferably from 30 to 200 mol% and most preferably from 50 to 150 mol%, in each case based on the starting material (a).

13. The process according to claim 9, **characterized in that** the palladium (II) salt and/or the palladium (II) complex (b1) is added in a concentration of from 0.01 to 20 mol%, preferably from 0.5 to 10 mol% and most preferably from 1 to 5 mol%, in each case based on the starting material (a).

14. The process according to claim 10, **characterized in that** the process is carried out at temperatures between 0°C and 100°C, preferably between 0°C and 50°C, and most preferably between 0°C and 25°C.

## Revendications

1. Une Procédé de préparation de monocétones macrocycliques insaturées comprenant les étapes suivantes
(a) mise à disposition de diènes macrocycliques avec une taille de cycle d'au moins 9 atomes de carbone ;
(b) mise en contact des matières premières de l'étape (a) avec
(b1) un sel de palladium (II) et/ou un complexe de palladium (II) ; et
(b2) un ligand bidentate contenant des atomes donneurs de N,N, N,O, N,S ou O,O, et
(b3) un agent oxydant ; et
(b4) un solvant ; et éventuellement
(b5) un co-catalyseur ; et éventuellement
(b6) un acide.

2. Procédé selon la revendication 1, **caractérisé en ce que** le solvant (b4) est un solvant aprotique polaire et est de préférence choisi dans le groupe constitué par les amides d'acides à chaîne ouverte et cycliques N,N-disubstitués, comme par exemple le diméthylformamide, diméthylacétamide, diéthylacétamide, diméthylpropio-namide, N-méthylpyrrolidone, nitriles aliphatiques, cycloaliphatiques ou aromatiques tels que l'acétonitrile, le propionitrile ou le benzonitrile ou éthers linéaires et cycliques, car-bonates cycliques et lactones.

3. Procédé selon la revendication 1 et/ou 2, **caractérisé en ce que** le sel de palladium (II) et/ou le complexe de palladium (II) (b1) sont choisis dans le groupe constitué par le chlorure de palladium, le bromure de palladium, H₂PdCl₄, Li₂PdCl₄, Na₂PdCl₄, K₂PdCl₄, (NH₄)₂PdCl₄, acétate de palladium, trifluoroacétate de palladium, benzoate de palladium, nitrate de palladium, sulfate de palladium, tétrafluoroborate de tétrakis(acétonitrile)palladium(Il) et/ou bis(trifluorométhane¬sulfonate) de tétrakis(acétonitrile)palladium(II).

4. Procédé selon l'une des revendications 1 à 3, **caractérisé en ce que** le sel de palladium (II) et/ou le complexe de palladium (II) (b1) forment avec le ligand bidentate (b2) un composé de palladium de formule (I) et/ou de formule (II) 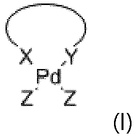
- dans laquelle X et Y représentent chacun indépendamment N,N, O,N ou O,O ; et
- où chaque Z représente indépendamment un halogène, un acétate, un triméthylacétate, un trifluorométhylacétate, MeCN, PhCN, NO₂, NO, un nitrate, un nitrite ou un sulfate ; 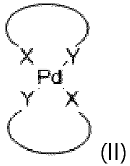
- où X et Y représentent chacun indépendamment O,O, N,N ou O,N.

5. procédé selon la revendication 4, **caractérisé en ce que** le composé de palladium est ajouté à une concentration de 0,01 à 25 % en moles, de préférence de 1 à 20 % en moles, de manière particulièrement préférée de 1 à 5 % en moles, dans chaque cas par rapport au matériau de départ (a).

6. Procédé selon l'une des revendications 1 à 5, **caractérisé en ce que** l'on utilise comme oxydant (b3) un gaz contenant de l'oxygène.

7. Procédé selon la revendication 6, **caractérisé en ce que** le gaz contenant de l'oxygène contient de l'oxygène à une concentration de 1 à 100 % en volume, de préférence de 5 à 100 % en volume et plus préférentiellement de 21 à 100 % en volume.

8. Procédé selon l'une quelconque des revendications 1 à 7, **caractérisé en ce que** le procédé est mis en oeuvre à des températures allant de 50°C à 120°C, de préférence de 60°C à 100°C, de manière la plus préférée de 70°C à 90°C.

9. Procédé selon la revendication 1, **caractérisé en ce que** le cocatalyseur (b5) et l'acide (b6) sont obligatoirement présents.

10. Procédé selon la revendication 9, **caractérisé en ce que**
(i) le co-catalyseur (b5) est choisi dans le groupe constitué par les benzoquinones, les naphtoquinones, les anthraquinones, l'acide molybdophosphorique, les acides molybda-tovanadatophosphoriques, les acides tungstomolybdophosphoriques, les acides tungstomolyvanadato¬phosphoriques, les complexes de phtalocyanine, FeSO₄, CuCI, CuCl₂, CuSO₄, VOSO₄, et
(ii) l'acide (b6) est un acide de Brönsted ou un acide de Lewis.

11. Procédé selon la revendication 10, **caractérisé en ce que** le co-catalyseur (b5) est ajouté à une concentration de 1 à 300% en moles, de préférence de 5 à 150% en moles et de manière la plus préférée de 21 à 100% en moles, dans chaque cas par rapport au matériau de départ (a).

12. procédé selon la revendication 10, **caractérisé en ce que** l'acide (b6) est ajouté à une concentration de 5 à 500% en moles, de préférence de 15 à 300% en moles, plus préférentiellement de 30 à 200% en moles et le plus préférentiellement de 50 à 150% en moles, respectivement par rapport au matériau de départ (a).

13. Procédé selon la revendication 9, **caractérisé en ce que** le sel de palladium (II) et/ou le complexe de palladium (II) (b1) est ajouté à une concentration de 0,01 à 20 % en moles, de préférence de 0,5 à 10 % en moles et de manière particulièrement préférée de 1 à 5 % en moles, respectivement par rapport au matériau de départ (a).

14. Procédé selon la revendication 10, **caractérisé en ce que** le procédé est mis en oeuvre à des températures comprises entre 0°C et 100°C, de préférence entre 0°C et 50°C, et plus préférentiellement entre 0°C et 25°C.
